# EUROPÄISCHE PATENTSCHRIFT

(11) **EP 1 690 505 B1**
(45) Veröffentlichungstag und Bekanntmachung des Hinweises auf die Patenterteilung: **20.08.2008**
(21) Anmeldenummer: 05100926.4
(22) Anmeldetag: 09.02.2005
(51) Int. Cl.: A61B 17/64, F16B 7/04

(54) **Einsatz für ein Klemmelement, Klemmelement mit einem solchen Einsatz und daraus gebildete Gelenkverbindung**
Insert for a clamping element, clamping element with such an insert and articulation composed thereof
Insert pour une borne, borne avec une tel insert et articulation composée de cela

(43) Veröffentlichungstag der Anmeldung: 16.08.2006
(73) Patentinhaber: Stryker Trauma SA, 2545 Selzach (CH)
(72) Erfinder: Thomke, Roland, 4512 Bellach (CH); Burgherr, Vinzenz, 3005 Bern (CH); Fankhauser, Damian, 3007 Bern (CH)
(74) Vertreter: Liebetanz, Michael

(56) Entgegenhaltungen:
- DE-A1- 3 823 746
- US-A- 5 888 221
- US-A- 6 080 153
- US-A1- 2004 044 344
- US-B1- 6 340 361

## Beschreibung

### Technisches Gebiet der Erfindung

Die Erfindung betrifft einen Einsatz für ein Klemmelement zum Klemmen eines stabförmigen Elementes einer Gelenkverbindung, insbesondere für ein Klemmelement einer Gelenkverbindung zur Stabilisierung von Knochenbrüchen. Weiterhin betrifft die Erfindung eine Gelenkverbindung mit zwei Klemmelementen und mit einem Verriegelungselement. Insbesondere betrifft die Erfindung einen Einsatz zur Optimierung der Fixierung von runden Stäben.

### Stand der Technik

Aus der US 6,616,664 und der EP 0 700 664 ist jeweils eine Gelenkverbindung bekannt, die aus vier einzelnen Klemmbackenelementen und einer zentralen Schraube besteht. Bei dieser Gelenkverbindung ist es möglich, einen oder zwei stabförmige Elemente in die entsprechenden Hohlräume seitlich einzuführen. Bei der EP 0 700 664 ist zwischen den beiden mittleren Klemmbackenelementen eine Feder angeordnet, gegen deren Federkraft es möglich ist, die stabförmigen Elemente einzuklipsen und so vor einer Blockierung der Gelenkverbindung diese an den stabförmigen Elemente festzuhalten. Bei der US 6,616,664 sind schmale, seitlich angeordnete Hebelarme vorgesehen, um seitlich eingelegte stabförmige Elemente vor einer Blockierung der Gelenkverbindung festzuhalten.

Diese genannten Gelenkverbindungen werden insbesondere bei rahmenartigen Systemen zur externen Behandlung von Knochenbrüchen eingesetzt, auch externer Fixateur genannt. Durch geeignete Materialpaarungen bei den Klemmen der Gelenkverbindungen und bei den Stäben, beispielsweise verschieden harte Stähle oder eine Kombination von Stahl zu Aluminium oder durch geeignete Oberflächenstrukturen von Innenoberfläche der Klemme und der Mantelfläche des Stabes kann erreicht werden, dass die stabförmigen Elemente nach dem Fixieren der Feststellschrauben ausreichend gegen Verdrehung und axiale Verschiebung gesichert sind.

Es ist interessant, zu versuchen, solche externen Fixateur in nicht metallischem Material, beispielsweise in KunststoffMaterialien, auszuführen, insbesondere um eine Gewichtsreduktion eines solchen Systems zu erreichen Falls die Baugruppen oder Teile einer solchen Klemme in Kunststoff-Spritzguss ausgeführt sind, besteht die Schwierigkeit, geeignete Materialpaarungen zu finden, mit welchen eine ausreichende Fixierung erreicht werden kann. Dies ist insbesondere deswegen ein Problem, weil sich viele Kunststoffe durch ein negatives Kriechverhalten auszeichnen, durch welches sich festgezogene Verbindungen nach und nach lösen bzw. ihre Spannkraft verlieren.

Als weiteres Problem stellt sich bei solchen Konfigurationen die hohe Druckbelastung der Kunststoffteile bei festgezogener Schraubverbindung dar, welche Belastung zu Spannungsrissen aufgrund unregelmässiger Belastung des Kunststoffgefüges führen können. Gefährlich ist dies insbesondere deshalb, da derartige Defekte erst nach einer gewissen Zeit auftreten und optisch nicht ohne weiteres ersichtlich sind.

Die US-A-5,888,221 zeigt einen Wirbelsäulenhaken mit den Merkmalen des Oberbegriffs des Anspruchs 1. Der Haken wird durch ein Blättchen als Einsatz über eine Schraube mit einem in den Kopfbereich des Hakens einlegbaren Stab fest verbunden.

Die US 2004/044344 A1 zeigt ein Klemmelement für einen Fixator, bei dem in den Fig. 15ff ein seitlicher Einsatz dargestellt ist. Dieser bildet eine Backe für die direkte Ausübung einer Klemmkraft auf einen in die Backe einlegbaren Stab über den konischen Schraubenhals der den Einsatz festsetzenden Schraube.

Die US-A-6,080,153 ist ein Mitglied der Patentfamilie zur oben genannten EP 0 700 664 und zeigt eine Gelenkverbindung für einen externen Fixator.

Ausgehend von diesem Stand der Technik ist es eine Aufgabe der vorliegenden Erfindung, einen Einsatz für ein Klemmelement anzugeben, welches das seitliche Einlegen von stabförmigen Elementen ermöglicht, und wobei das Klemmelement doppelt verwendet direkt als Gelenkverbindung einsetzbar ist, wobei die oben genannten Elemente des Klemmelementes aus einem nicht metallischen Material beispielsweise aus Kunststoff bestehen können, ohne dass dies die Sicherheit der sich ergebenden Gelenkverbindung in einem externen Fixateur beeinträchtigt.

Ferner ist es eine Aufgabe der vorliegenden Erfindung, ein einfaches für den Einsatz von solchen Einsätzen konzipiertes Klemmelement anzugeben.

Ausgehend vom bekannten Stand der Technik liegt der Erfindung weiterhin die Aufgabe zugrunde, eine verbesserte Gelenkverbindung anzugeben.

Ziel der Erfindung ist es mit anderen Worten somit, eine geeignete Massnahme anzubieten, welche einen optimalen und möglichst direkten Kraftschluss zwischen der Verbindungsklemme und den stabförmigen Elementen ermöglicht, der konstant bleibt und gleichzeitig zu keiner Überbelastung der Kunststoffteile führt und somit Strukturschäden vermeiden hilft. Es soll die dauerhafte Stabilität von mehrheitlich in Kunststoff ausgeführten Rahmenkonfigurationen für externe Fixateure gewährleistet werden, das heisst die von den bisherigen metallischen Konstruktionen bekannte Zuverlässigkeit gesichert werden. Zudem soll es sich um eine leicht herstellbare und günstig montierbare Konstruktion handeln, um ein kostengünstiges Produkt zu erhalten.

### Zusammenfassung der Erfindung

Diese Aufgabe wird erfindungsgemäss für einen Einsatz der eingangs genannten Art mit den kennzeichnenden Merkmalen des Anspruchs 1 gelöst. Ein entsprechendes Klemmelement ist durch die Merkmale des Anspruchs 5 gekennzeichnet. Eine erfindungsgemässe Gelenkverbindung ist durch die Merkmale des Anspruchs 7 gekennzeichnet.

Insbesondere ermöglicht ein erfindungsgemässer Einsatz eine direkte Kraftübertragung von der Schraube oder einem anderen Verriegelungselement auf den Stab zur Entlastung des Kunststoffbauteils.

Bei anderen Ausführungsformen ist es auch möglich, die Einsätze als umspritzte Einlegeteilen zu erreichen. Dabei würden metallische Teile in die Spritzgussform eingelegt und danach mit Kunststoff umspritzt. Die ist eine gängige Technik, welche jedoch einen aufwendigen und dementsprechend teuren Produktionsprozess erfordert (robotergesteuertes Einlegen der Metallteile oder manuelle Bestückung). Ein Nachteil einer solchen Lösung ist, dass umspritzte Einlegeteile ebenfalls den erwähnten Kriechphänomenen unterworfen sind.

Weitere vorteilhafte Ausführungsbeispiele sind in den Unteransprüchen niedergelegt.

### Kurze Figurenbeschreibung

Die Erfindung wird nun unter Bezugnahme auf die Zeichnungen an Hand von Ausführungsbeispielen beispielhaft näher beschrieben. Es zeigen:
- Fig. 1: eine geschnittene Seitenansicht einer Gelenkverbindung mit zwei Klemmelementen mit Einsätzen nicht nach einem Ausführungsbeispiel der Erfindung ausgebildet,
- Fig. 2: eine perspektivische Ansicht der Fig. 1 mit einem eingelegten Stab,
- Fig. 3: eine perspektivische Ansicht des Einsatzes aus Fig. 11,
- Fig. 4: eine Unteransicht des Einsatzes gemäss Fig. 3,
- Fig. 5: eine Seitenansicht des Einsatzes gemäss Fig. 3,
- Fig. 6: eine perspektivische Ansicht eines anderen Einsatzes, nicht nach einem Ausführungsbeispiel ausgebildet,
- Fig. 7: eine Unteransicht des Einsatzes gemäss Fig. 6,
- Fig. 8: eine Seitenansicht des Einsatzes gemäss Fig. 6,
- Fig. 9: eine perspektivische Ansicht eines weiteren Einsatzes nicht nach einem Ausführungsbeispiel ausgebildet,
- Fig. 10: eine Unteransicht des Einsatzes gemäss Fig. 9,
- Fig. 11: eine Seitenansicht des Einsatzes gemäss Fig. 9,
- Fig. 12: eine geschnittene Seitenansicht einer Gelenkverbindung mit zwei Klemmelementen mit Einsätzen gemäss einem Ausführungsbeispiel der Erfindung,
- Fig. 13: eine perspektivische Ansicht der Gelenkverbindung nach Fig. 13 mit einem eingelegten Stab,
- Fig. 14: eine perspektivische Ansicht des Einsatzes gemäss dem Ausführungsbeispiel,
- Fig. 15: eine Unteransicht des Einsatzes gemäss Fig. 14,
- Fig. 16: eine Seitenansicht des Einsatzes gemäss Fig. 14,
- Fig. 17: eine Draufsicht auf den Einsatz gemäss Fig. 14,
- Fig. 18: eine perspektivische Ansicht des weiteren Einsatzes nicht nach einem Ausführungsbeispiel ausgebildet,
- Fig. 19: eine Seitenansicht des Einsatzes gemäss Fig. 18,
- Fig. 20: eine perspektivische Ansicht eines weiteren Einsatzes nicht nach einem Ausführungsbeispiel ausgebildet,
- Fig. 21: eine Seitenansicht des Einsatzes gemäss Fig. 20,
- Fig. 22: eine Unteransicht des Einsatzes gemäss Fig. 20,
- Fig. 23: eine perspektivische Ansicht einer Gelenkverbindung mit Einsätzen nach Fig. 20 mit einem eingelegten Stab, und
- Fig. 24: eine geschnittene Seitenansicht der Gelenkverbindung nach Fig. 23.

### Ausführliche Beschreibung der bevorzugten Ausführungsbeispiele

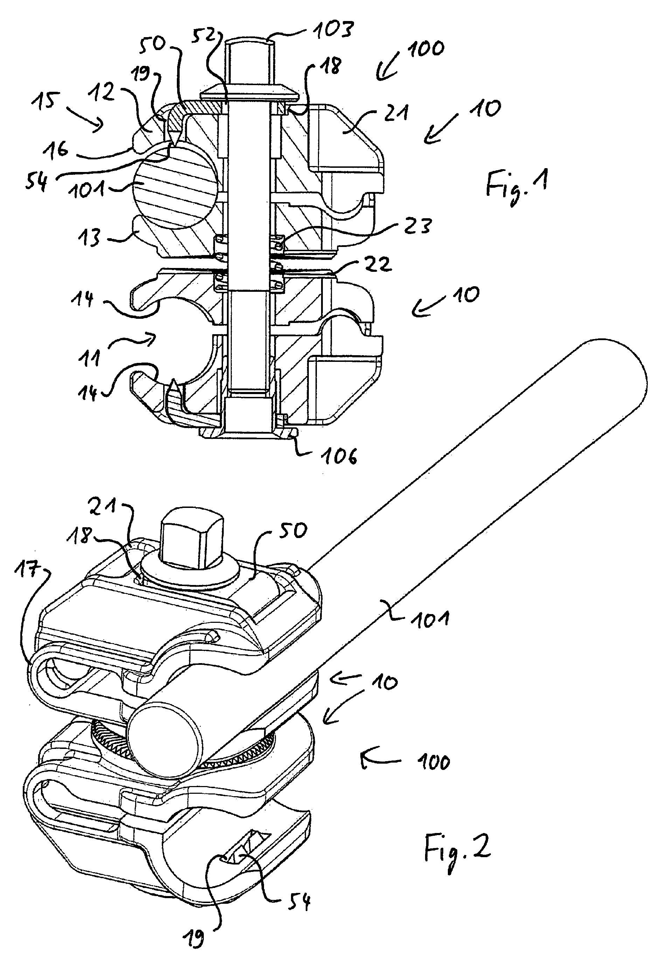

Die Fig. 1 zeigt eine geschnittene Seitenansicht einer Gelenkverbindung 100 mit zwei Klemmelementen 10 mit Einsätzen 50, nicht gemäss einem ersten Ausführungsbeispiel der Erfindung ausgebildet.

Jedes Klemmelement 10 verfügt über zwei gegenüberliegende und einen Hohlraum 11 zur Aufnahme eines stabförmigen Elementes aufweisende Klemmbacken 12 und 13. Die Klemmbacken 12 und 13 verfügen an ihren freien Enden 15 jeweils über eine quer verlaufende Nut 14, die zusammen den Hohlraum 11 aufspannen. An den freien Enden 15 sind die Aussenkanten 16 der aufeinander zugewandten Seite der Klemmbacken 12 und 13 gerundet, um das Einschieben eines stabförmigen Elementes von der Seite her zu vereinfachen. Gegenüber dem Hohlraum 11 und den freien Enden 15 ist ein in der Fig. 2 zu erkennendes Scharniermittel 17 vorgesehen, welches die Klemmbacken 12 und 13 einstückig miteinander verbindet. Eine Schraube 103, die durch die Klemmelemente 10 hindurchtritt und in eine Mutter 106 einschraubbar ist, schliesst das Gelenkelement 100 und verspannt in dieses einlegbare Stäbe 101. Dabei handelt es sich bei der Schraube-Mutter-Verbindung um ein Verriegelungselement, welches auch über Hebel und andere Elemente realisierbar ist.

Die Klemmelemente 10 weisen im Mittelbereich einen vollen Materialquerschnitt auf, die zwei seitliche Querrippen 21 ausbilden, die insbesondere in dem oberen Bereich der Klemmbacke 12 stark ausgeprägt sind. Der Bereich zwischen den Querrippen 21 ist zur Vorderkante 16 hin bis auf eine in der Draufsicht rechteckige Einsatzaufnahme 18 ausgenommen. Die Einsatzaufnahme 18 verfügt zentral in dem Klemmelement 10 zur Aufnahme der Schraube 103 über eine durchgehende Bohrung. Die Rippen 21 können je nach Breite des Einsatzes 50 auch vollkommen an der Aussenseite des Klemmelementes verlaufen. Die Einsatzaufnahme 18 kann auch in einem Abstand zu den Rippen 21 in einer zweiten Stufe bestehen.

In der unteren Klemmbacke 13 münden die besagten Querrippen 21 in einem Ringflansch 22, der beispielsweise über einen flachen vertieften ringförmigen Absatz verfügen kann, an den sich eine gewichts-, materialsparende und für die Spritzgussherstellung vorteilhafte Ausnehmung anschliessen kann, in deren Mitte eine Bohrung vorgesehen ist. Diese durchgehende Bohrung ist fluchtend mit der oben genannten durchgehenden Bohrung in der oberen Klemmbacke 12 ausgerichtet. Sie verläuft beim Klemmelement 10 senkrecht zur Achse des Hohlraums 11 und parallel zur Rückseite des Scharniermittels 17. Sie könnte aber auch schräg verlaufen. Der Ringflansch 22 kann Träger von Eingriffsmitteln wie radialen Zähnen sein, um ein Verdrehen der Klemmelemente 10 gegeneinander zu verhindern. Es kann auch ein getrenntes Verdrehelement zum Einsatz zwischen den beiden Klemmelementen 10 vorgesehen sein.

In der Darstellung der Fig. 1 wird das Gelenkelement 100 aus zwei Klemmelementen 10 gebildet. Die Klemmelemente 10 sind beispielsweise für einen Stab mit einem Durchmesser von beispielsweise 12 Millimetern vorgesehen. Dann hat die Öffnung an den freien Enden im Ruhezustand einen Durchmesser von beispielsweise 8 Millimetern. Wenn nun das obere Klemmelement 10 für einen Stab mit 4 bis 6 Millimetern Durchmesser vorgesehen sein soll, dann hätte die Öffnung an den freien Enden 15 im Ruhezustand einen Durchmesser von beispielsweise 2 Millimetern.

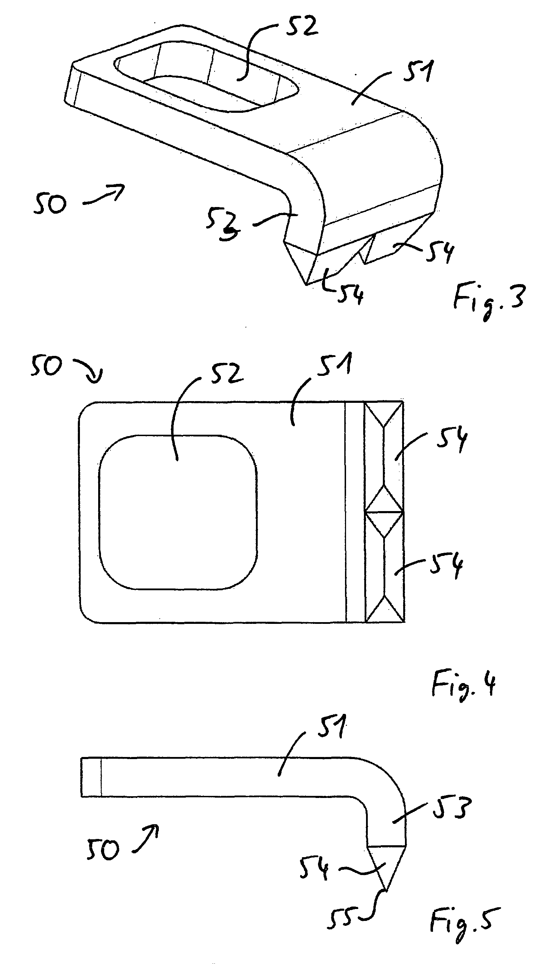

In die Einsatzaufnahme 18 ist ein Einsatz 50 eingesetzt, der aus einem L-förmigen Profil besteht, welches in den Fig. 3 und weiteren genauer beschrieben werden wird. In der oberen Klemmbacke 12 ist in der Einsatzaufnahme 18 eine durchgehende Schlitzbohrung 19 vorgesehen, durch die die Spitzen des L-förmigen Profils des Einsatzes 50 bis in den Bereich des Hohlraums 11 ragen können; die Schlitzbohrung 19 mündet in der Nut 14, vorteilhafterweise mittig in den Nut 14. Sie können beispielsweise immer um 1 Millimeter in die Nut 14 hineinragen, weil die Profilfläche 51 des Einsatzes 50 im Presssitz in der Klemmbacke 12 sitzt.

Die Fig. 2 zeigt eine perspektivische Ansicht der Fig. 1 mit einem eingelegten Stab 101. Gleiche Merkmale in den Zeichnungen werden jeweils mit gleichen Bezugzeichen dargestellt. In dem unteren Klemmelement 10 sind der Durchbruch der Schlitzbohrung 19 und zwei Zähne des unteren Einsatzes 50 zu erkennen, welches durch die Mutter 106 gehalten wird. Die Verbindung der Klemmelemente 10 wird dabei über eine zwischen diese eingelegte Druckfeder 23 auseinandergedrückt.

Die Fig. 3 zeigt eine perspektivische Ansicht des Einsatzes 50 gemäss Fig. 1, Fig. 4 eine Unteransicht auf diesen Einsatz 50 und Fig. 5 eine Seitenansicht des Einsatzes 50. Der Einsatz 50 ist in der Draufsicht im wesentlichen eine rechteckige Platte 51 mit einer Langloch-Bohrung 52. Im Querschnitt zeigt sich der Einsatz 50 als L-förmiges Plättchen mit einer langen Seite 51 und einer kürzeren Eingriffsseite 53. Das freie Ende der kürzeren Eingriffsseite 53 endet in hier zwei Zähnen 54, die im Querschnitt eine Prismenform aufweisen. Der Einsatz 50 dient als ein Einlegeteil im Kunststoffteil des Klemmelementes 10, welches unter dem Schraubenkopf 103 bzw. unter der Mutter 106 in das Kupplungselement 10 eingelegt wird. Vorzugsweise handelt es sich bei dem Einsatz 50 hierbei um ein Stanzprägeteil. In dieser Ausführung wird das Metallteil 50 bei Bezugnahme auf die Fig. 1 und 2 auf der Aussenseite des Gelenkelementes 100 angebracht. Der Einsatz 50 ist derart gestaltet, dass er sich mit einer abgewinkelten Vorderseite 53 durch den Durchbruch oder Schlitzbohrung 19 im Klemmelement 10 direkt in ein rundes stabförmiges Element 101 einkrallen kann und so zu einer Materialdeformation bzw. Einkerbung im stabförmigen Element 101 führt. Dadurch wird ein optimaler Kraftschluss zwischen den Elementen 10 (über Einsatz 50) und einem Stab 101 erreicht.

Der Vorteil besteht insbesondere darin, dass der Kraftschluss über das Einlegeteil 50 direkt zwischen der Schraube 103 (oder Mutter 106) und dem stabförmigen Element 101 zustande kommt und das Kunststoffbauteil 10 des Kupplungselementes 100 nur noch als Führungselement dient. Dadurch wird es erheblich entlastet und kann auf diese Führungsfunktion hin optimal gestaltet werden. Die Eingriffskante 55 der Zähne 54 ist dabei im wesentlichen axial in Richtung des Stabes 101 ausgerichtet und verhindert insbesondere hervorragend eine Rotation.

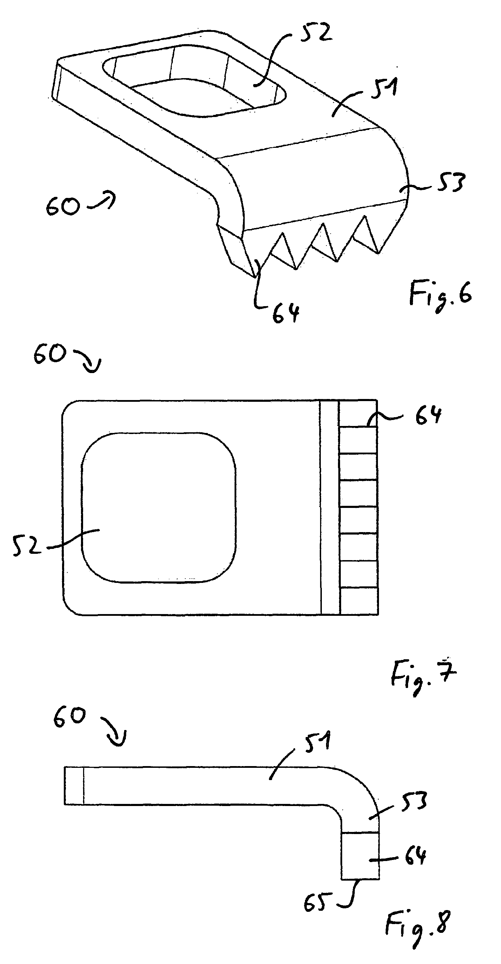

Die Fig. 6 zeigt eine perspektivische Ansicht eines anderen Einsatzes 60, nicht gemäss einem Ausführungsbeispiel ausgebildet. Fig. 7 zeigt eine Unteransicht des Einsatzes 60 gemäss Fig. 6 und Fig. 8 eine Seitenansicht des Einsatzes 60 gemäss Fig. 6. Der Einsatz 60 ist im wesentlichen sehr ähnlich zum Einsatz 50 mit dem Unterschied, dass hier vier Zähne 64 vorgesehen sind, und dass die jeweilige Eingriffskante 65 der Zähne 64 quer zur longitudinalen Richtung eines Stabe 101 steht, in den sie eingreifen kann. Damit steht die Eingriffskante 65 der Zähne 64 im wesentlichen quer zur Richtung des Stabes 101 und verhindert insbesondere hervorragend eine axiale Verschiebung des Klemmelementes 10 auf dem Stab 101.

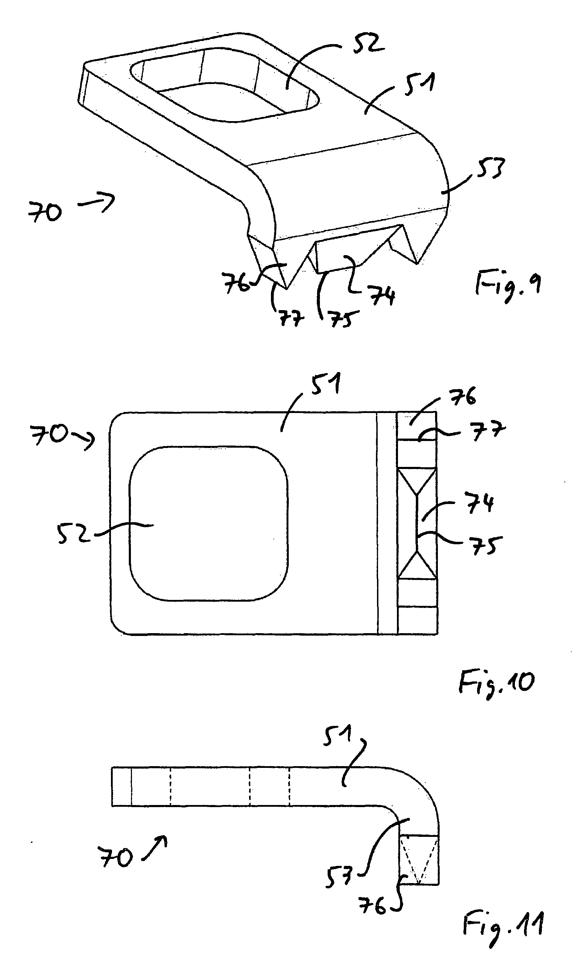

Die Fig. 9 zeigt eine perspektivische Ansicht eines Einsatzes 70, nicht gemäss einem Ausführungsbeispiel ausgebildet, Fig. 10 eine Unteransicht des Einsatzes 70 gemäss Fig. 9 und Fig. 11 eine Seitenansicht des Einsatzes gemäss Fig. 9. In dieser Ausführungsvariante sind im Prinzip die ersten und zweiten Ausführungsbeispiele kombiniert. Der Einsatz 70 verfügt über Längszähne 74 mit einer Eingriffskante 75 gegen Rotationen und über Querzähne 76 mit einer Eingriffskante 77 gegen axiale Verschiebungen. Hier ist ein zentraler Längszahn 74 und zwei seitlich von diesem angeordnete Querzähne 76 vorgesehen; es könnten auch zwei oder mehr Längszähne 74 und nur auf einer Seite ein Querzahn 76 oder - vorteilhafterweise - symmetrisch zwei oder mehr Querzähne 76 vorgesehen sein. Wesentlich ist bei diesem Ausführungsbeispiel , dass es rechtwinklig zueinander ausgerichtete Zähnen 74 bzw. 76 sind, um sowohl die Längsverschiebung der Stäbe 101, wie auch deren Rotation sicher zu unterbinden.

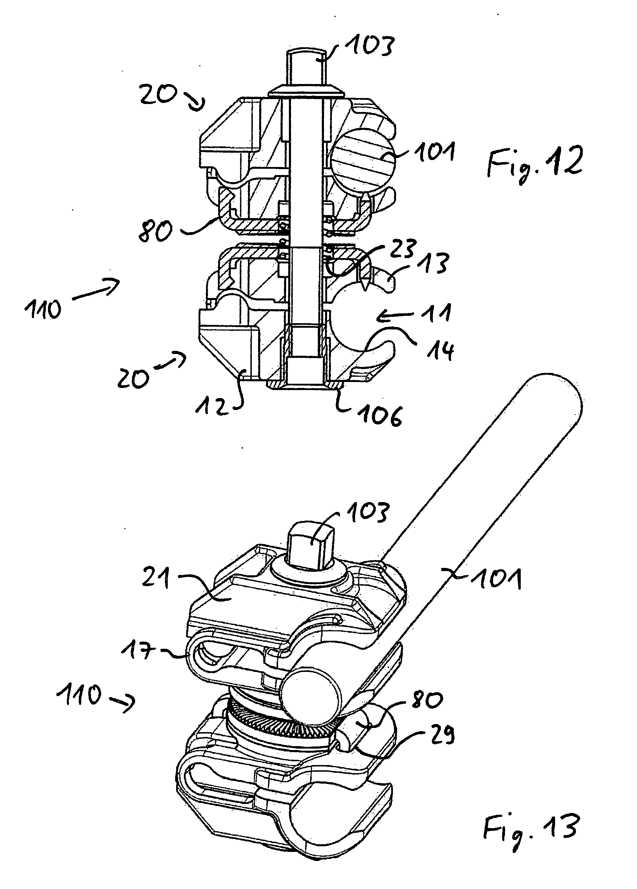

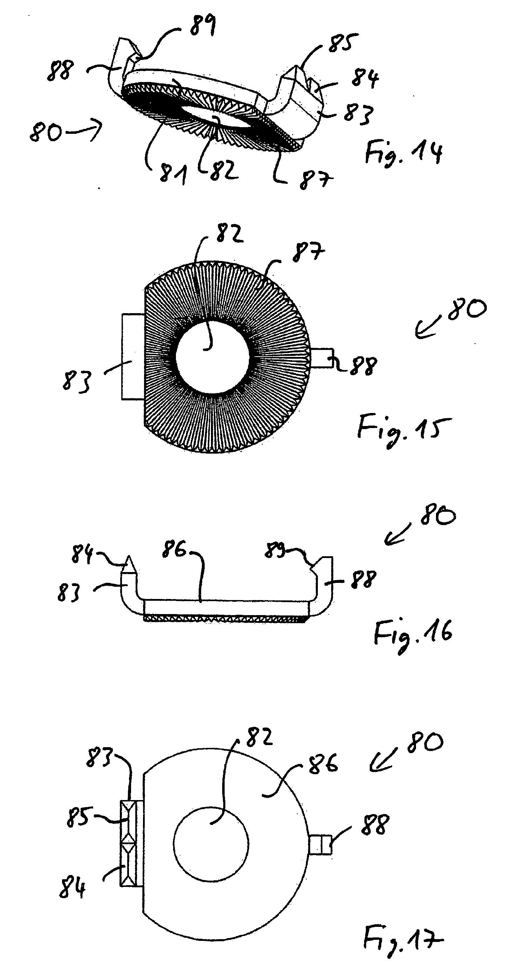

Die Fig. 12 zeigt eine geschnittene Seitenansicht einer zweiten Gelenkverbindung 110 mit zwei Klemmelementen 20 mit Einsätzen 80 gemäss einem Ausführungsbeispiel der Erfindung und die Fig. 13 zeigt eine perspektivische Ansicht dieses Ausführungsbeispiels gemäss der Erfindung mit einem eingelegten Stab 101. Die Klemmelemente 20 entsprechen einem anderen Ausführungsbeispiel gegenüber den Klemmelementen 10, da sie für die Aufnahme der Einsätze 80 entsprechend modifiziert sind, die im Zusammenhang mit den Fig. 14 bis 17 beschrieben werden. Die Fig. 14 zeigt eine perspektivische Ansicht des Einsatzes 80 gemäss dem Ausführungsbeispiel, die Fig. 15 eine Unteransicht davon, die Fig. 16 eine Seitenansicht und schliesslich die Fig. 17 eine Draufsicht auf den Einsatz 80 gemäss Fig. 14.

Die untere Klemmbacke 13 verfügt auf ihrer Unterseite (im Gegensatz zum in den Fig. 1 und 2 dargestellten geriffelten Flansch 22) über eine flache Auflagefläche, auf die die Innenseite 86 des Einsatzes 80 aufliegt.

Ferner verfügt das Klemmelement 20 über eine der Schlitzbohrung 19 entsprechende Schlitzbohrung 29, die entsprechend in der Nut 14 der Klemmbacke 13 mündet. Der Einsatz 80 ist U-förmig ausgestaltet und verfügt, ähnlich zum Einsatz 50 über eine Plattenfläche 81, die hier rund ausgestaltet ist und auf der Seite der Eingriffseite 83 abgeflacht ist. Beim dargestellten Ausführungsbeispiel sind zwei Zähne 84 mit Eingriffskanten 85 vorgesehen. Es ist klar, dass auch andere Konfigurationen entsprechend den obigen zweiten und dritten Ausführungsbeispielen realisiert werden können.

Auf der der Eingriffseite 83 gegenüberliegenden Seite ist ein Verriegelungsbalken 88 vorgesehen, der im wesentlichen parallel zu der Eingriffseite 83 verläuft. Der Verriegelungsbalken 88 verläuft in einem parallel zur die Schraube aufnehmenden Bohrung verlaufenden Schlitz der Klemmelemente 20 und verfügt über eine Verriegelungsnase 89, die sich in der Tiefe des Schlitzes in das Material des Klemmelementes 20 eindrücken kann.

In dieser Ausführungsvariante kommen die beiden Einsätze 80 direkt zwischen den beiden zum Beispiel Kunststoffteilen der Klemmelemente 20 direkt aufeinander zu liegen und übernehmen zusätzlich die Funktion der Verdrehsicherung 87 der Klemmelemente 20. Die Kunststoffteile verfügen in diesem Fall über keine Verzahnung an der Unterseite. Vorzugsweise wird der Einsatz als Stanz-Prägeteil hergestellt. Die radialen Zähne der Verdrehsicherung 87 greifen beim Festziehen der Schraube 103 ineinander und um die Verdrehsicherung zu verbessern, verfügt der Einsatz zusätzlich über einen Verriegelungsbalken 88, der in die Klemmbacke 13 des Kunststoffteiles 20 eingreift.

Zudem kann dieser Einsatz 80 mit Haken 89 und Eingriffsseite 83 als Klammer oder Schnapper ausgebildet werden, was die Montage vereinfacht, da die Teile so an das Klemmelement 20 angeklickt werden können. Auch bei dieser Variante können die in die Stäbe 101 eingreifenden Zähne in verschiedensten Formen ausgebildet sein, von denen die dargestellten Ausführungsbeispiel nur eine vorteilhafte Auswahl bilden. Es können beispielsweise statt der Zähne 54, 64, 74, 76, 84 jeweils oder zusätzlich auch rundkonische Spitzen vorgesehen sein. Es sind jeweils Eingriffselemente. Als Eingriffselement wird hier ein jedes solches Mittel angesehen, welches man als ein Mittel zur Erzeugung von hohen Reibungskräften bezeichnen kann. Dies kann einfach eine raue Oberfläche aufweisen, es können eine oder ganz viele teppichartige kleine Spitzen vorliegen oder das Eingriffselement kann weitere in Beschreibung und Ansprüchen genannte Ausführungsformen annehmen.

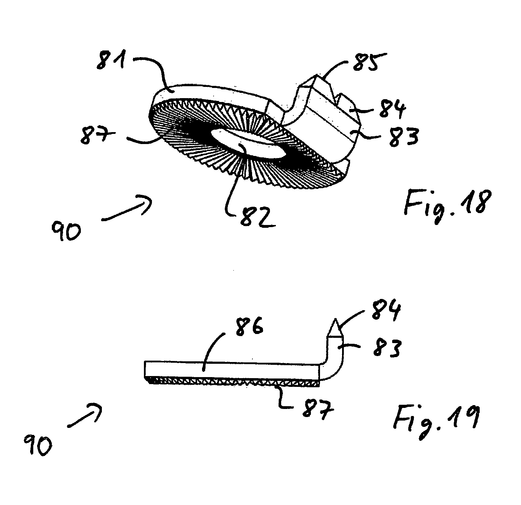

Die Fig. 18 zeigt eine perspektivische Ansicht eines Einsatzes 90, nicht gemäss einem Ausführungsbeispiel ausgebildet, und Fig. 19 eine Seitenansicht dieses Einsatzes 90 gemäss Fig. 18. Der einzige Unterschied zwischen diesen Einsatzen liegt darin, dass der Einsatz 90 über keinen Verriegelungsbalken 88 verfügt. Damit sind die Zähne 84, die sich bei einem Fixieren des Gelenkelementes in die Stäbe 101 einkrallen, die einzigen Elemente, die gleichzeitig das Drehen der Kunststoffklemmen 10 oder 20 verhindern. Damit wird die Verdrehsicherung 87 nur einseitig abgesichert und nutzt insbesondere den Formschluss in der Schlitzbohrung 29 nur asymmetrisch.

Es ist klar, dass auch der Einsatz 50 gemäss Fig. 3 mit einem Verriegelungsbalken 88 ausgestaltet werden kann, wenn eine entsprechende Ausnehmung in der Klemmbacke 12 vorgesehen ist.

Es ist direkt erkennbar, dass die beiden für die inneren Klemmbacken 13 vorgesehenen Einsätze 80 beziehungsweise 90 über eine Rundbohrung 82 verfügen, während die für die äusseren Klemmbacken 12 vorgesehenen Einsätze 50, 60 beziehungsweise 70 rechteckige Bohrungen oder Langbohrungen aufweisen. Dies liegt daran, dass zum Einlegen der Stäbe 101 die Klemmbacken 12 und 13 auseinander zudrücken sind. Dabei bewegen sich die Klemmbacken 12 um einen Schwenkpunkt des Scharnierteils 17 und damit relativ zur Schraube 103 oder Mutter 106, womit es notwendig ist, dass ein entsprechender Freiraum für den Weg der Schraube 103 oder Mutter 106 vorhanden ist. Anstelle der geschlossenen Bohrung kann auch ein U-förmiger Schlitz vorgesehen sein, der ein Klemmelement 103, 106 mindestens teilweise umgibt.

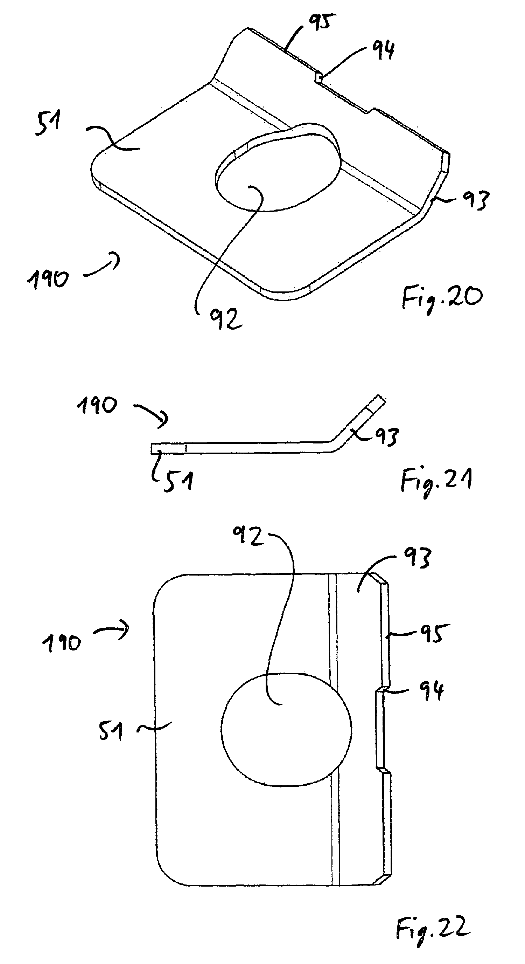

Die Fig. 20 zeigt eine perspektivische Ansicht eines Einsatzes 190, nicht gemäss einem Ausführungsbeispiel ausgebildet. Die Fig. 21 zeigt eine Seitenansicht des Einsatzes 190 gemäss Fig. 20 und die Fig. 22 eine Unteransicht.

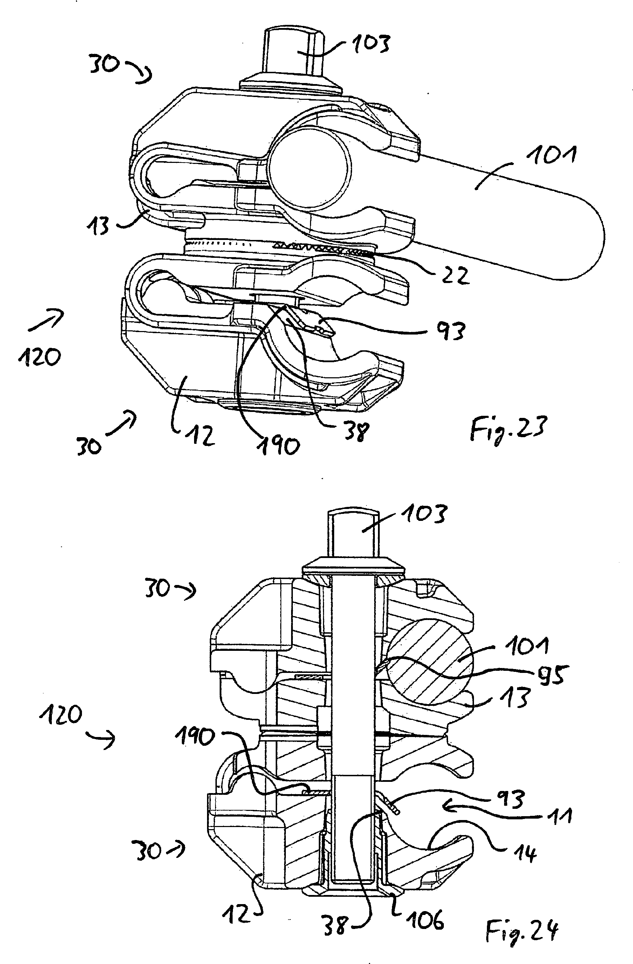

Der Einsatz 190 ist rechteckig mit abgerundeten Ecken ausgestaltet und verfügt ähnlich zum Einsatz 50 über eine Plattenfläche 51, die auf der einen Seite schräg in die Eingriffseite 93 übergeht. Dies kann beispielsweise durch eine Stanzung erreicht werden. Der Winkel zwischen Fläche 51 und der Eingriffseite 93 kann beispielsweise 45 Grad betragen, aber auch andere Werte vorteilhafterweise zwischen 30 und 60 Grad sind möglich. In der Fläche 51 ist ein Langloch 92 angeordnet, welches seine Längsrichtung quer zur Stanzlinie der Eingriffseite 93 ausgerichtet hat. Der kleinere Durchmesser des Langloches 92 entspricht im wesentlichen dem Durchmesser der vorgesehenen Schraube 103 für das Klemmelement 30 nach Fig. 23. Das Langloch 92 hat einen Bereich, der sich bis in die Schräge der Eingriffsseite 93 hinein erstreckt. Dies ist von Vorteil, da so bei dem Klemmelement 30 die Nut 14 für den Stab 101 dicht an die quer verlaufende Schraube 103 herangebracht werden kann. Der sich dadurch verkürzende Bereich der Eingriffsseite 93 ist von Vorteil, da zwei Stäbe 101 somit dicht über die Schraube 103 miteinander starr verkoppelt werden und wenig Last auf die das Gelenkelement 120 aufbauenden Klemmelemente 30 wirkt.

Beim dargestellten Ausführungsbeispiel sind zwei seitliche Zähne 94 mit Eingriffskanten 95 vorgesehen. Man kann dies auch als eine Eingriffskante 95 beschreiben, die im mittleren Bereich zurückgesetzt ist. Es ist klar, dass auch andere Eingriffs-Konfigurationen entsprechend den obigen ersten bis fünften Ausführungsbeispielen realisiert werden können.

Auf der der Eingriffseite 93 gegenüberliegenden Seite ist hier kein Verriegelungsbalken vorgesehen, der senkrecht zur Fläche 51 verlaufen würde. Ein solches zum Verriegelungsbalken 88 ähnliches Verriegelungselement ist natürlich denkbar, um die Verriegelung zu unterstützen.

In dieser Ausführungsvariante kommen die beiden Einsätze 190 direkt zwischen den beiden Klemmbacken 12 und 13 eines jeden Klemmelementes 30 zu liegen. Die Klemmbacke 12 verfügt dafür über eine Schräge 38, an die die Eingriffsseite 93 anliegen kann. Die Schraube 103 durchstösst dabei die Öffnung 92, die als Langloch ausgestaltet ist, um eine Bewegung des Einsatzes 190 quer zur Ausrichtung 101 zu erlauben.

Die Fig. 23 zeigt eine perspektivische Ansicht einer Gelenkverbindung 120 mit zwei Klemmelementen 30 mit Einsätzen 190 nach Fig. 20 mit einem eingelegten Stab 101 und Fig. 24 eine geschnittene Seitenansicht dieser Gelenkverbindung 120 nach Fig. 23. Die Klemmelemente 30 entsprechen einem anderen Ausführungsbeispiel gegenüber den Klemmelementen 10, da sie für die Aufnahme der Einsätze 190 entsprechend modifiziert sind.

Im unteren Teil der Fig. 24 ist zu erkennen, dass der Einsatz 190 auf Grund der Schwerkraft auf der Klemmbacke 12 aufliegt. Dabei kann die Eingriffsseite 93 an der Schräge 38 der Klemmbacke 12 anliegen, sie muss es aber nicht, da das Langloch 92 eine Bewegung des Einsatzes 190 gegenüber der Schraube 103 gestattet.

Die Eingriffskante 95 ragt in den Hohlraum 11. Die Eingriffskante 95 ist dabei in ihrer Höhe zwischen den Klemmbacken 12 und 13 ungefähr mittig ausgerichtet. Bei einem Einsetzen des Stabes 101 durch Eindrücken desselben über die Kanten der Klemmbacken 12 und 13, was im oberen Bereich der Fig. 24 dargestellt ist, kommt der Stab 101 in der Nut 14 zu liegen. Dabei verdrängt er die Eingriffsseite 93 gegen den Rand der Nut 14 und insbesondere gegen die Schräge 38. Dann greift die Eingriffskante 95 in den Stab 101 ein.

Auch bei dieser Variante können die in die Stäbe 101 eingreifenden Zähne in verschiedensten Formen ausgebildet sein, von denen die dargestellten Ausführungsbeispiel nur eine vorteilhafte Auswahl bilden. Es können beispielsweise statt der Zähne 54, 64, 74, 76, 84 jeweils oder zusätzlich auch rundkonische Spitzen vorgesehen sein. Es sind jeweils Eingriffselemente.

Bei allen dargestellten Ausführungsbeispielen bildet der Einsatz eine abgewinkelte Unterlagsscheibe, die direkt im Kraftschluss auf einen Stab 101 eingreift. Damit werden die Klemmelemente 10, 20 oder 30 nur noch als Führungs- und Distanzhalteteil verwendet; sie werden somit weit weniger belastet. Dies gestattet eine nochmals grössere Materialauswahl bei den Klemmelementen 10, 20 30. Dabei greift der Einsatz 50, 60, 70, 80, 90 oder 190 auf der anderen Seite in das Verriegelungselement, hier 103, 106 ein.

Die Einsätze 50, 60 und 70 sind mit einer Ausnehmung 18 dargestellt, in die sie sich einfügen. Diese Ausnehmung 18 kann insbesondere so ausgestaltet sein, dass die Einsätze 50, 60 und 70 darin kein Spiel haben, sondern sich in einem Presssitz einfügen. Sie können dann nicht herausfallen und bilden für den Benutzer eine einstückige Einheit. Das gleiche kann durch entsprechende Umformung der unteren Klemmbacke 13 auch für den Einsatz 80 erreicht werden. Damit kann man zudem in Bezug auf die Verdrehsicherung einen ähnlichen Effekt wie mit dem Verriegelungsbalken erreichen, insbesondere, wenn der Einsatz 80 nicht rund sondern eckig ausgestaltet wird, wobei auf der der flachen Seite 86 gegenüberliegenden Seite die gegen eine Verdrehung sichernden Merkmale vorgesehen sind, beispielsweise radiale Nuten.

Schliesslich können die Einsätze auch als umspritzte Teile in einem Klemmelement einstückig vorgesehen sein.

Die in den Zeichnungen dargestellten Ausführungsbeispiele beziehungsweise die in der vorliegenden Beschreibung erwähnten Merkmale von verschiedenen Ausführungsbeispielen sind nicht notwendigerweise als voneinander unabhängige Ausführungsformen zu verstehen. Vielmehr ist es möglich, dass jedes in einem der Ausführungsbeispiele beschriebene Merkmal mit einem oder mehreren beliebigen anderen Merkmalen aus anderen Ausführungsbeispielen kombiniert werden kann, so dass sich andere, nicht wörtlich oder unter Bezugnahme auf Zeichnungen beschriebene Ausführungsbeispiele ergeben, die im Rahmen der vorliegenden Ansprüche in den beanspruchten Schutzbereich fallen.

### Bezugszeichen

- 10: Klemmelement
- 11: Hohlraum
- 12: Klemmbacke
- 13: Klemmbacke
- 14: quer verlaufende Nut
- 15: freies Ende
- 16: Aussenkante
- 17: Scharniermittel
- 18: Einsatzaufnahme
- 19: Schlitzbohrung
- 20: Klemmelement ( Ausführungsbeispiel)
- 21: Querrippen
- 22: Ringflansch
- 23: Druckfeder
- 30: Klemmelement
- 38: Schräge
- 50: Einsatz
- 51: rechteckige Platte
- 52: Langlochbohrung
- 53: Eingriffsseite
- 54: Zahn
- 55: Eingriffskante
- 60: Einsatz
- 64: Zahn
- 65: Eingriffskante
- 70: Einsatz
- 74: Zahn
- 75: Eingriffskante
- 76: Zahn
- 77: Eingriffskante
- 80: Einsatz
- 81: Plattenfläche
- 82: Rundbohrung
- 83: Eingriffsseite
- 84: Zahn
- 85: Eingriffskante
- 86: Innenseite
- 87: Verdrehsicherung
- 88: Verriegelungsbalken
- 89: Verriegelungsnase
- 90: Einsatz
- 92: Langloch
- 93: Eingriffseite
- 94: Zahn
- 95: Eingriffskante
- 100: Gelenkelement
- 101: stabförmiges Element
- 103: Schraube
- 106: Mutter
- 110: Gelenkelement
- 120: Gelenkelement
- 190: Einsatz

## Patentansprüche

1. Einsatz (80) für ein Klemmelement (20) mit zwei Backen (12, 13) zum Klemmen eines stabförmigen Elementes (101) mit Hilfe eines Verriegelungselementes (103, 106), wobei der Einsatz ein Profilstück mit zwei freien Enden ist, **dadurch gekennzeichnet, dass** der Einsatz eine im Querschnitt U-förmige Scheibe ist, an deren einem freien Ende mindestens ein Eingriffselement ( 84) zum Eingriff in das stabförmige Element (101) vorgesehen ist, dass eine Bohrung (82) zwischen den beiden freien Enden zum Eingriff mit dem Verriegelungselement (103, 106) vorgesehen ist, und dass an der dem Eingriffselement ( 84) gegenüberliegenden freien Ende ein Verriegelungselement (88, 89) zum Eingriff in das Klemmelement (20) vorgesehen ist.

2. Einsatz nach Anspruch 1, **dadurch gekennzeichnet, dass** das oder die Eingriffselemente (84) in jeweils einer oder in einer Vielzahl von Spitzen und/oder in einer Eingriffskante (85) auslaufen und/oder eine raue Oberfläche bilden.

3. Einsatz nach Anspruch 2, **dadurch gekennzeichnet, dass** mindestens zwei Eingriffselemente (74, 76) vorgesehen sind, die zwei senkrecht zueinander ausgerichtete Eingriffskanten (75, 77) aufweisen.

4. Einsatz nach einem der Ansprüche 1 bis 3, **dadurch gekennzeichnet, dass** im Bereich der besagten Bohrung (82) zum Eingriff mit dem Verriegelungselement (103, 106) auf der von den Eingriffselementen (84) abgewandten Seite des Einsatzes ein Verdrehsicherungsmittel (87) vorgesehen ist.

5. Klemmelement (20), das über zwei gegenüberliegende und einen seitlich offenen Hohlraum (11) zur seitlichen Aufnahme eines stabförmigen Elementes (101) bildende Klemmbacken (12, 13) verfügt, **dadurch gekennzeichnet, dass** es über eine quer zur besagten Aufnahme verlaufende Durchgangsbohrung (19, 29) zwischen einer Aussenseite einer Klemmbacke (12, 13) und der den Hohlraum (11) begrenzenden Nut (14) sowie über eine parallel zur Durchgangsbohrung (19, 29) verlaufende Ausnehmung verfügt, um durch die Durchgangsbohrung (19, 29) hindurch das besagte eine freie Ende mit dem oder den Eingriffselementen (84) eines Einsatzes nach einem der Ansprüche 1 bis 4 aufzunehmen, wobei die besagte Ausnehmung zur Aufnahme des Verriegelungselementes (88, 89) des besagten Einsatzes dient.

6. Klemmelement nach Anspruch 5, **dadurch gekennzeichnet, dass** der Einsatz über Ausnehmung und Durchgangsbohrung (19, 29) in das Klemmelement (20) einklipsbar ist.

7. Gelenkelement (110)
- mit zwei Klemmelementen, wobei mindestens eines (20) nach einem der Ansprüche 5 oder 6 ausgebildet ist, wobei die Klemmelemente mit ihren inneren Klemmbacken (13) aufeinander ausgerichtet übereinander angeordnet sind,
- mit mindestens einem in das besagte Klemmelement nach einem der Ansprüche 5 oder 6 eingesetzten Einsatz (80) nach einem der Ansprüche 1 bis 4.

## Claims

1. Insert (80) for a clamping element (20), having two jaws (12, 13) for clamping a rod-shaped element (101), using a locking element (103, 106), wherein the insert is a profiled piece having two free ends, **characterized in that** the insert is a U-shaped disk in cross-section, at one free end of which at least one engagement element (84) for engagement into the rod-shaped element (101) is provided, wherein a bore (82) is provided between the two free ends for engagement with the locking element (103, 106), and that a locking element (88, 89) for engagement in the clamping element (20) is provided on the side that lies opposite the engagement element (84).

2. Insert according to claim 1, **characterized in that** the engagement element(s) (84) run into one or a plurality of points and/or into an engagement edge (85), in each instance, and/or form a rough surface.

3. Insert according to claim 2, **characterized in that** at least two engagement elements (74, 76) are provided, which have two engagement edges (75, 77) that are oriented perpendicular to one another.

4. Insert according to one of claims 1 to 3, **characterized in that** a rotation locking means (87) is provided in the region of the said bore (82), for engagement with the locking element (103, 106), on the side of the insert that faces away from the engagement elements (84).

5. Clamping element (20) that has two clamping jaws (12, 13) that lie opposite one another and form a laterally open cavity (11) for lateral accommodation of a rod-shaped element (101), **characterized in that** it has a passage bore (19, 29) that runs crosswise to the said accommodation, between an outer side of a clamping jaw (12, 13) and the groove (14) that delimits the cavity (11), as well as it has a recess that runs parallel to the passage bore (19, 29) in order to accommodate the said free end, with the engagement element(s) (84) of an insert according to one of claims 1 to 4, through this bore (19, 29), wherein the recess is provided to accommodate the locking bar (88, 89) of said insert.

6. Clamping element according to claim 5, **characterized in that** the insert can be clipped into the clamping element (20), via the recess and the passage bore (19, 29).

7. Articulated element (110)
- having two clamping elements, whereby at least one (20) is configured according to one of claims 5 or 6, whereby the clamping elements are disposed above one another, with their inner clamping jaws (13) oriented towards one another,
- having at least one insert (80) according to one of claims 1 to 4 to be inserted into a clamping element according to one of claims 5 or 6.

## Revendications

1. Insert (80) pour un élément de serrage (20), avec deux mâchoires (12, 13) pour serrer un élément en forme de barre (101), en utilisant un élément de verrouillage (103, 106), où l'insert étant une pièce profilée avec deux bouts libres, **caractérisé en ce que** l'insert est un disque en forme de U en section transversale, où, à un bout libre, au moins un élément interposant (84) est prévu pour engager l'élément en forme de barre (101), où un alésage (82) est prévu entre les deux bouts libres pour engager l'élément de verrouillage (103, 106), et **en ce que**, pour engager l'élément de serrage (20), un élément de verrouillage (88, 89) est prévu sur un côté qui est opposé à l'élément interposant (84).

2. Insert selon la revendication 1, **caractérisé en ce que** les éléments interposant (84) se terminent dans une pointe ou dans une pluralité de points et/ou dans un bord interposant (85) et/ou forment une surface rêche.

3. Insert selon la revendication 2, **caractérisé en ce qu'**au moins deux éléments interposants (74, 76) sont prévus, qui possèdent deux bords interposants (75, 77) qui sont orientés perpendiculairement l'un par rapport à l'autre.

4. Insert selon l'une quelconque des revendications 1 à 3, **caractérisé en ce qu'**un moyen anti-rotation (87) est prévu dans la région dudit alésage (82) pour engager l'élément de verrouillage (103, 106) sur le côté de l'insert qui est orienté à l'opposé des éléments interposants (84).

5. Elément de serrage (20) qui comprend deux mâchoires (12, 13) qui sont disposées l'une opposée à l'autre et forment une cavité (11) qui est latéralement ouverte pour un accueil latéral d'un élément en forme de barre (101), **caractérisé en ce qu'**il possède un alésage de passage (19, 29) qui est disposé transversalement à cet accueil entre une face extérieure d'une mâchoire (12, 13) et la rainure (14) qui délimite la cavité (11), ainsi qu'un creux qui est disposé parallèlement audit alésage de passage (19, 29) pour accueillir ledit bout libre comprenant l'élément interposant (84) d'un insert selon une quelconque des revendications 1 à 4, à travers de cet alésage de passage (19, 29), où ledit creux est prévu d'accueillir l'élément de verrouillage (88, 89) dudit insert.

6. Elément de serrage selon la revendication 5, **caractérisé en ce que** l'insert peut être attaché par clip dans l'élément de serrage (20) par le creux et l'alésage de passage (19, 29).

7. Elément d'articulation (110),
- avec deux éléments de serrage, où au moins un (20) est configuré selon l'une des revendications 5 ou 6, où les éléments de serrage sont disposés l'un sur l'autre avec leur mâchoires (13) intérieures orientées l'une vers l'autre,
- possédant au moins un insert (80) selon l'une quelconque des revendications 1 à 4, pour être engagé dans un élément de serrage selon l'une quelconque des revendications 5 à 6.
